# EUROPÄISCHE PATENTSCHRIFT

(11) **EP 3 558 508 B1**
(45) Veröffentlichungstag und Bekanntmachung des Hinweises auf die Patenterteilung: **30.03.2022**
(21) Anmeldenummer: 17703342.0
(22) Anmeldetag: 25.01.2017
(51) Int. Cl.: B01J 13/14, A01N 25/28, A61K 8/11, A61K 9/50, C09B 67/02, C11D 3/50, F28D 20/02

(54) **MIKROKAPSELN**
MICROCAPSULES
MICROCAPSULES

(30) Priorität: 22.12.2016 WO PCT/EP2016/082399
(43) Veröffentlichungstag der Anmeldung: 30.10.2019
(73) Patentinhaber: Symrise AG, 37603 Holzminden (DE); Papierfabrik August Koehler SE, 77704 Oberkirch (DE)
(72) Erfinder: JURISCH, Claus, 77654 Offenburg (DE); HORN, Michael, 77654 Offenburg (DE); MEIER, Claudia, 77839 Lichtenau (DE); BERTRAM, Ralf, 37603 Holzminden (DE); OTT, Patrick, 37603 Holzminden (DE)
(74) Vertreter: Fabry, Bernd
(86) Internationale Anmeldenummer: PCT/EP2017/051481
(87) Internationale Veröffentlichungsnummer: WO 2018/114056

(56) Entgegenhaltungen:
- WO-A1-2006/050638
- US-A1- 2004 195 711
- DATABASE WPI Week 199118 Thomson Scientific, London, GB; AN 1991-129339 XP002773353, -& JP H03 68948 A (CANON KK) 25. März 1991 (1991-03-25)
- DATABASE WPI Week 199012 Thomson Scientific, London, GB; AN 1990-088101 XP002773354, -& JP H02 42444 A (FUJI PHOTO FILM CO LTD) 13. Februar 1990 (1990-02-13)

## Beschreibung

### GEBIET DER ERFINDUNG

Die Erfindung befindet sich auf dem Gebiet von Kapseln mit einer hohen Wirkstoff- bzw. Wirksubstanzbeladung, sowie Zubereitungen für den Endverbraucher, die die Kapseln enthalten.

### TECHNOLOGISCHER HINTERGRUND

Verkapselungen oder Einkapselungen von Wirkstoffen, insbesondere von Aroma- bzw. Duftstoffen oder kosmetischen oder pharmazeutischen Wirkstoffen oder Agrochemikalien sind Stand der Technik und bieten oftmals die Möglichkeit, das verkapselte bzw. eingekapselte Material zu stabilisieren und vor Reaktionen mit dem Medium zu schützen, um so die Wirkung des Wirkstoffes zu erhalten und kontrolliert frei zu setzen.

Neben makroskopischen Partikeln mit Durchmessern im Bereich bis hin zu 1 cm, sind insbesondere Mikrokapseln von Interesse. Hierunter werden vom Fachmann sphärische Partikel mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit Polymeren umhüllte feindisperse flüssige oder feste Phasen, bei deren Herstellung sich die Polymere nach Emulgierung und z.B. Koazervation oder Grenzflächenpolymerisation auf dem einzuhüllenden Material niederschlagen. Die Hülle solcher Mikrokapseln kann aus natürlichen, halbsynthetischen oder synthetischen Materialien bestehen.

### STAND DER TECHNIK

Die Verkapselung von Wirkstoffen unter Einsatz von Gelatine und Polysacchariden, speziell Gummi Arabicum ist Gegenstand zahlreicher Schutzrechte. Die ältesten Druckschriften stammen aus den Jahren 1958 bis 1974, nämlich US 3,041,288; JP 50 027826 A und JP 51 013387 A.

JP 368948 A (CANON) offenbart mit Farbstoffen beladene Mikrokapseln, bei denen die Hülle aus Polymeren, nämlich Harnstoff-Formaldehyd- oder Melamin-FormaldehydHarzen in Kombination mit einer Phenolkomponente bestehen. Es werden also Polymere statt Monomere eingesetzt, außerdem werden keine Parfümöle verwendet.

JP 242444 A (FUJI) betrifft ein Verfahren zur Einarbeitung eines Reduktionsmittels in die fotoempfindliche Außenschicht einer Mikrokapsel. Es wird nicht ersichtlich, wie die Mikrokapsel aufgebaut ist; sie enthält jedenfalls kein Parfümöl.

Beispiele für Mikrokapseln des Stands der Technik sind folgende Handelsprodukte (in Klammern angegeben ist jeweils das Hüllmaterial): Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose); Unicerin C30 (Lactose, mikrokristalline Cellulose, Hydroxypropylmethylcellulose), Kobo Glycospheres (modifizierte Stärke, Fettsäureester, Phospholipide), Softspheres (modifiziertes Agar-Agar) und Kuhs Probiol Nanospheres (Phospholipide) sowie Primaspheres und Primasponges (Chitosan, Alginate) und Primasys (Phospholipide). Sowie Kapseln aus synthethischen Polymeren Micronal^{®} (BASF), Mikrokapseln 500 und 560 (Koehler SE), Folco Smartcaps^{®}, Enfinit^{™}, Ensensa^{™}

### AUFGABE DER ERFINDUNG

Die klassischen Verkapselungsverfahren im Lebensmittelbereich sind zumeist wasserbasiert und liefern somit auch nur wasserlösliche Partikel. Da aber nahezu alle Lebensmittel Wasser enthalten, können die üblichen Technologien, wie z.B. Sprühtrocknung, Sprühgranulation oder Extrusion die geforderte Freisetzung bei Erhitzung des Lebensmittels oder beim Verzehr nicht oder nur sehr begrenzt leisten.

Die in Mikrokapseln eingeschlossenen Substanzen und Wirkstoffe werden allgemein als Kernmaterial bezeichnet. Durch die Auswahl geeigneter Wandmaterialien können die physikalischen und chemischen Eigenschaften von Mikrokapseln gezielt beeinflusst werden. Die Handhabung und Lagerung der Produkte in Pulverform macht sie ausgesprochen anwenderfreundlich. Je nach Verwendung verschiedener Wand- und Kernmaterialien ergeben sich vielseitige Einsatzmöglichkeiten.

Beispiele für Inhaltsstoffe und Einsatzgebiete von Mikrokapseln liegen in Bereichen von Duftstoffen (Duftmarketing, Duftlacke), Aromen, Farbstoffen, z.B. bei Durchschreibepapieren (erste industrielle Anwendung der Mikroverkapselung, Patent 1953), Leuchtfarben, Ölen und Schmierstoffen (Schmierung bei mechanischer Beanspruchung), Klebstoffen (kleben unter Druckeinwirkung), Lösungsmitteln, Reinigungsmitteln, Desinfektionsmitteln, Konservierungsmitteln, Waschmittel (Enzymen), Pharmazeutika, Nahrungsergänzungsmitteln (verzögerte Freisetzung, Retardierung), Pestiziden (bessere, weniger gesundheitsgefährdende Handhabung), Flammschutzmitteln, optischen Aufhellern, reaktiven Kunststoffen (Epoxidharze, Polyurethane) sowie selbstheilenden Oberflächenbeschichtungen und massiven Materialien, Bohrhilfsmitteln, Latentwärmespeichern, Korrosionsschutz, Prozesshilfsmitteln wie Katalysatoren, Vernetzern oder Rheologiehilfsmitteln, Entschäumern und Tensiden.

Oftmals problematisch bei den Partikeln im Stand der Technik ist die Kapselhülle. Insbesondere wenn eine hohe Beladung von Aroma- oder Duftstoffe erreicht werden soll, diffundieren Aroma- oder Duftstoffe häufig bei der Lagerung in extraktiven Anwendungsformulierungen (z.B. Tensidlösungen) aus der Kapselhülle, bevor sie zur Anwendung kommen.

Die Aufgabe der vorliegenden Erfindung hat daher darin bestanden, stabile Kapseln, die Wirkstoffe bzw. Substanzen enthalten und die eine Lagerstabilität von mindestens 8 Wochen in der Anwendungsformulierung haben, zu erzeugen. Ferner war es Aufgabe der vorliegenden Erfindung Kapseln zu entwickeln, die eine variable Beladung ermöglichen, und auch eine hohe Wirkstoffbeladung ermöglichen, so dass die Kapseln breit möglichst angewendet werden können, d.h. die Kapseln können Wirkstoffe aus unterschiedlichen Bereichen wie Wasch- und Reinigungsmitteln, Klebstoffen, Beschichtungszusammensetzungen, Agrochemikalien, aber auch kosmetischen und pharmazeutischen Bereichen verkapseln und lassen sich entsprechend in den unterschiedlichsten Produkten einarbeiten.

### BESCHREIBUNG DER ERFINDUNG

Ein erster Gegenstand der Erfindung sind Mikrokapseln, umfassend oder bestehend aus
(a) einem Kern, enthaltend mindestens ein, zwei oder mehrere Wirkstoffe, und
(b) einer Hülle,
wobei das Wandmaterial der Hülle geformt wird aus einem oder mehreren Aminoplasten, die gebildet werden aus
(i) mindestens einem Harnstoffderivat oder Melaminderivat,
(ii) einer Carbonylverbindung, und
(iii) mindestens einer Aminophenolkomponente der Formel (la) und/oder (Ib), 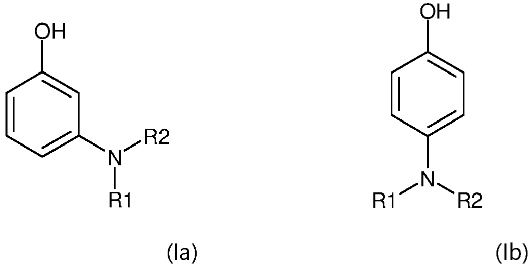 worin R1 und R2 unabhängig voneinander Wasserstoff, Methyl oder Ethyl darstellen, mit den Maßgaben, dass
   - die Wirkstoffe Duftstoffe oder Parfümöle darstellen, von denen mindestens einer bei 25 °C flüssig sein muss, und
   - die Harnstoffderivate oder Melaminderivate ausgewählt sind aus der Gruppe, die gebildet wird von 2,4,6-Triamino-1,3,5-triazin (Melamin), Tetrahydroimidazo[4,5-*d*]imidazole-2,5(1*H*,3*H*)-dion (Glycoluril), Benzoguanamin, Acetoguanamin, Adipound Glutaroguanamin oder einer Mischung daraus.

Überraschenderweise wurde gefunden, dass Mikrokapseln gemäß vorliegender Erfindung besonders lagerstabil in z.B. tensidhaltigen Anwendungsformulierungen sind. Ein weiterer Vorteil der erfindungsgemäßen Kapseln ist ihre Stabilität, die es ermöglicht, die Kapseln in den unterschiedlichsten Bereichen einzusetzen, um die gewünschten, je nach Anwendungsbedarf, unterschiedlichen Wirkstoffe und Wirkstoffsubstanzen in das entsprechende Medium einzubringen und bei Bedarf freizusetzen.

### Wirkstoffe für die Verkapselung

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie bei-spielsweise Zibet und Castoreum. Typische synthetische Riechstoffverbindungen sind Pro-dukte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ▪-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Ber-gamotteöl, Dihydromyrcenol, Lilial, Lyral, Citronellol, Phenylethylalkohol, ▪-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylgly-colat, Cyclovertal, Lavandinöl, Muskateller Salbeiöl, ▪-Damascone, Geraniumöl Bourbon, Cyclohexylsalicylat, Vertofix Coeur, Iso-E-Super, Fixolide NP, Evernyl, Iraldein gamma, Phe-nylessigsäure, Geranylacetat, Benzylacetat, Rosenoxid, Romilllat, Irotyl und Floramat allein oder in Mischungen, eingesetzt.

Ferner besteht eine weitere bevorzugte Ausführungsform der vorliegenden Erfindung in Kapseln, die die vorgenannten Wirkstoffe bzw. Wirkstoffsubstanzen umfassen, aber auch die die im Folgenden genannten Inhaltsstoffe bzw. Wirkstoffe bzw. Wirkstoffsubstanzen für kosmetische und Wasch- und Reinigungsmitteln, umfassen.

### Herstellverfahren

Die erfindungsgemäßen Mikrokapseln lassen sich mittels eines Verfahrens herstellen umfassend die folgenden Schritte:
A) Bereitstellung einer Prä-Emulsion, umfassend Stabilisatoren und Wandbildner, sowie die zu verkapselnden Wirkstoffe;
B) Initiierung der Kondensation durch Temperatur-und/ oder pH-Wert-Änderung, optional durch Alkoholzusatz oder Aussalzen;
C) Nachhärtung durch
   (c1) Zugabe einer Dispersion, enthaltend mindestens ein Harnstoffderivat oder Melaminderivat oder
   (c2) eines entsprechenden Vorkondensates aus Harnstoff und/oder Melamin und einem Aldehyd,
   wobei die Harnstoffderivate oder Melaminderivate ausgewählt sind aus der Gruppe, die gebildet wird von 2,4,6-Triamino-1,3,5-triazin (Melamin), Tetrahydroimidazo[4,5-*d*]imidazole-2,5(1*H*,3*H*)-dion (Glycoluril), Benzoguanamin, Acetoguanamin, Adipo- und Glutaroguanamin oder einer Mischung daraus, sowie
   Zugabe von Aminophenolkomponenten (la) und/oder (Ib), vorzugsweise in wässriger Form, bei Temperaturen von 50°C bis 100°C;
D) Zugabe von Harnstoff, vorzugsweise in wässriger Form oder als Feststoff;
E) Abkühlen der Reaktionsmischung; und gegebenenfalls
F) Sprühtrocknung oder Sprühgranulierung der erhaltenen Kapseln.

Überraschenderweise wurde gefunden, dass mittels des vorliegenden Herstellungsverfahrens besonders lagerstabile Mikrokapseln erhalten werden, die zudem wenig Formaldehyd enthalten. Bevorzugt ist die Konzentration an freien Aldehyden hierbei unter 400 ppm, besonders bevorzugt unter 300 ppm, ganz besonders bevorzugt unter 100 ppm. Die so hergestellten Mikrokapseln lassen sich demgemäß entsprechend in unterschiedlichen Produkten einarbeiten.

Üblicherweise entsteht bei der Herstellung der Mikrokapseln bezogen auf die Gesamtzusammensetzung weniger als 5 Gew.-% Formaldehyd. Bevorzugt liegt die Grenze von freien Formaldehyd durch das vorliegende Herstellungsverfahren im Bereich von 0,001 bis 5 Gew.%, bevorzugt von 0,01 bis 3 Gew.%, besonders bevorzugt von 0,01 bis 1 Gew.% basierend auf die Gesamtzusammensetzung einer Mikrokapsel.

### Verkapselung

Wandbildner und Stabilisatoren werden unter Rühren in Wasser gelöst. Die Lösung wird auf eine Temperatur im Bereich von 10 bis 100 °C, bevorzugt von 30 bis 90 °C eingestellt. Danach wird das Kernmaterial zugesetzt und in dieser Mischung emulgiert. Dabei gilt, dass mit stärkerer Rührleistung und längerer Reaktionszeit kleinere Kapseln gebildet werden und umgekehrt. Als weitere Zusatzstoffe kommen hier Säuren, wie Essigsäure, Ameisensäure, Zitronensäure oder auch Mineralsäuren, wie z.B. Salz- oder Schwefelsäure in Betracht, mit denen der pH-Wert der Lösung im sauren Bereich bei etwa 3 bis 5 gehalten wird. Da die Ansätze dazu neigen können, Schaum zu entwickeln, kann beispielsweise handelsüblicher Silikonentschäumer zugesetzt werden.

### Härtung bzw. Vernetzung der Kapseln

Die Kapseln weisen noch eine flexible Hülle auf, die keine sonderliche Stabilität besitzt und daher auch nicht die gewünschte Diffusionsdichte erreicht. Zu diesem Zweck wird eine Härtung bzw. Vernetzung der Hülle durchgeführt. Zur Härtung wird eine wässerige Melamin-Dispersion zugegeben und bei etwa 60 bis etwa 70 °C über etwa 30 min. bis etwa 1h gerührt. Danach wird auf ca. 80°C erwärmt und eine wässrige Aminophenollösung zugegeben und bei etwa 80°C bis etwa 90 °C über etwa 30 min. bis etwa 1h gerührt. Anschließend wird ein Harnstoffderivat in Form einer Lösung oder fest zugesetzt und wieder bei etwa 80°C bis etwa 90 °C über etwa 30 min. bis etwa 1h gerührt. Danach wird abgekühlt.

Vorzugsweise weisen die erfindungsgemäßen Kapseldispersionen eine sehr hohe Wirkstoffbeladung auf, die je nach Anwendungsbedarf variabel eingestellt wird. Bevorzugt weisen die erfindungsgemäßen Kapseln demgemäß eine Wirkstoffbeladung von 10 bis 60 Gew.%, bevorzugt von 20 bis 45 Gew.%, ganz besonders bevorzugt von 25 bis 40 Gew.%, auf, bezogen auf die Gesamtzusammensetzung der Kapseldispersion. Die Wirkstoff- bzw. Wirksubstanzbeladung ist abhängig von der Endapplikation der Kapseln und variiert dementsprechend je nach Einsatzgebiet. Die Beladungsmenge kann je nach Anwendungsbedarf entsprechend variiert und eingestellt werden. Die vorgenannten Beladungsmengenbereiche sind als mögliche Beispielgrößen zu verstehen, und sollen keine Einschränkung für die herstellbare Beladungsmengen darstellen.

Vorzugsweise können die erfindungsgemäßen Kapseln einen mittleren Durchschnittsdurchmesser von 1 bis 1000 µm, bevorzugt von 2 bis 80µm, aufweisen. Die Kapselgröße kann je nach Anwendungsbedarf entsprechend variiert und eingestellt werden. Demgemäß sind die vorgenannten Kapselgrößenbereiche als mögliche Beispielgrößen zu verstehen, und sollen keine Einschränkung für die herstellbaren Kapselgrößen darstellen.

In der vorliegenden Anmeldung wird der Begriff Kapsel mit dem Begriff Partikel gleichgestellt. Beide Begriffe sind äquivalent und untereinander austauschbar zu verstehen.

Zusätzlich kann ein Wandmaterial gewählt werden, welches als eine zweite Hülle um die vorliegenden Mikrokapseln formt. Es kann aus einem oder mehreren Polymeren aufgebaut sein, die ausgewählt sind aus natürlichen, halbsynthetischen, synthetischen Polymermaterial oder Mischungen daraus.

Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse.

Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol, Aminoplaste, Phenoplaste oder Polyvinylpyrrolidon.

Als Aminophenolkomponente wird bei der Härtung der vorliegenden Mikrokapseln die Verbindung (la) oder (Ib) eingesetzt oder eine Mischung aus (la) und (Ib), wobei im Fall von einer Mischung aus (la) und (Ib) das Verhältnis der Komponenten (Ia):(Ib) von 10:1 bis 1:5 beträgt. Bevorzugt ist die Aminophenolkomponente für die Herstellung der vorliegenden Mikrokapseln 3-Aminophenol.

### GEWERBLICHE ANWENDBARKEIT

Ein Vorteil der erfindungsgemäßen Kapseln, in der das Harnstoffderivat oder Melaminderivat und die genannten Aminophenolkomponenten zur Bildung der vorliegenden Mikrokapseln verwendet werden, ist ihr gutes Rückhaltevermögen, d.h. dass die erfindungsgemäßen Kapseln mindestens 30% bis 70%, bevorzugt mindestens 40% bis 60% Rückhaltung des Kapselinhaltes nach einer Lagerung in einer tensidhaltigen Anwendungsformulierung von 8 Wochen bei einer Temperatur von 45°C, aufweisen, bevorzugt nach einer Lagerung von 12 Wochen 20% bis 60%, bevorzugt 30% bis 50%, aufweist.

Bevorzugt werden die erfindungsgemäßen Kapseln zur Herstellung von pharmazeutischen oder kosmetischen Produkten oder Wasch- und Reinigungsmitteln verwendet. Vorzugsweise sind pharmazeutische und kosmetische Produkte, insbesondere geeignet zur Anwendung auf der Haut. Bevorzugt sind hierbei sowohl kosmetische Produkte als auch pharmazeutische Mittel in Form von Salben, Cremes, Lotionen, Gele und Pasten und Sprays.

Vorzugsweise ist unter eine Salbe, Creme, Lotion, Gel und Paste eine halbfeste streichfähige Zubereitung zu verstehen, die zum Auftragen auf die Haut geeignet sind.

Solche Zubereitungen können beispielsweise auf Basis von einer wässrigen (hydrophilen) und einer öligen bzw. fetten (lipophilen) Komponente bestehen, von der die eine emulsionsartig in der anderen verteilt ist.

Es können ebenfalls hydrophile Cremes vom O/W-Typ oder lipophile Cremes vom W/O-Typ sein. Daneben gibt es Cremes, die weder eindeutig dem O/W- noch dem W/O-Typ zuordenbar sind, die aus gelartig, kohärent ineinander verteilter lipophiler und hydrophiler Phase bestehen (amphiphile Creme). Auch Strukturen einer Mehrfachemulsion vom Typ W/O/W-Emulsion sind möglich. Hier liegt die innere Phase wiederum in Form einer Emulsion vor. In die innere Ölphase sind nochmals kleinste Wassertröpfchen eingelagert. Dieser Emulsionstyp soll die Vorteile von W/O-Emulsionen und O/W-Emulsionen in sich vereinen.

Weitere Zubereitungen sind bevorzugt Salben, die in der Regel eine halbfeste und homogen aussehende Zubereitung ist, und die geeignet ist zur Anwendung auf der Haut (z. B. als Wundsalbe) oder auf den Schleimhäuten. Salben dienen zumeist der lokalen Wirkstoffapplikation oder der Pflege und dem Schutz der Haut oder Schleimhäute. Vorzugsweise besteht eine Salbe aus einer hydrophoben oder hydrophilen Grundlage aus natürlichen oder synthetischen Stoffen und kann ein einphasiges (z. B. Vaseline) oder mehrphasiges (z.B. Wasser-in-ÖI) System sein.

Weiterhin können die erfindungsgemäßen Mikrokapseln verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendes-infektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom ÖI-in-Wasser-, vom Wasser-in-ÖI-und vom Wasser-in-ÖI-in-Wasser-Typ wie z.B. Hautcremes- und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haar-wässern, Haarcremes und -lotionen, Deodorantien und Antiperspirantien wie z.B. Achsel-sprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Bevorzugt werden die erfindungsgemäßen Kapseln in Wasch- und Reinigungsmittel (abgekürzt als WSR-Mittel) verwendet. WSR-Mittel im Sinne der vorliegenden Erfindung, können in fester Form als Pulver, Granulate, Tabletten und dergleichen, aber auch flüssig, gelförmig oder pastös vorliegen. Es handelt es sich vorzugsweise um Waschmittel, die sowohl für manuelle oder maschinelle Wäsche, insbesondere von Textilien geeignet sind. Es kann sich auch um Wasch- oder Reinigungsmittel für den industriellen Bereich oder für den Haushaltsbereich handeln. Reinigungsmittel können beispielsweise auch zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff oder Metall, in Haushalt und Gewerbe in Frage.

Die WSR-Mittel können weitere handelsübliche Bestandteile aufweisen, wie beispielsweise Tenside, Gerüststoffe, Bleichmittel, Bleichmittelaktivatoren, Verdickungsmittel, Enzyme, Elektrolyte, pH-Stellmittel, Farb- und Duftstoffe, Schauminhibitoren, Antiredepositionsmittel, optische Aufheller, Vergrauungsinhibitoren, Knitterschutzmittel, antimikrobielle Wirkstoffe, Konservierungsmittel, Antioxidantien, Antistatika, UV-Adsorber, Schwermetallkomplexbildner und dergleichen.

Ein weiterer Gegenstand der vorliegenden Erfindung sind Wasch- und Reinigungsmittel, Kosmetische Zubereitungen (speziell Körperpflegemittel), Parfümzusammensetzungen Agrochemikalien oder Klebstoffe, die die erfindungsgemäßen Mikrokapseln enthalten bzw. die Verwendung der Mikrokapseln zur Herstellung dieser Produkte.

### WASCH- UND REINIGUNGSMITTEL

### Gerüststoffe

Als Gerüststoffe bzw. Builder, die in den flüssigen Wasch- und Reinigungsmitteln enthalten sein können, die verkapselt werden können sind insbesondere Silikate, Aluminiumsilikate (insbesondere Zeolithe), Carbonate, organische Co-builder, Phosphate, Salze organischer Di- und Polycarbonsäuren sowie Mischungen dieser Stoffe zu nennen.

Geeignete kristalline, schichtförmige Natriumsilikate besitzen die allgemeine Formel NaMSiₓO₂ₓ₊₁*H2O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl beta - als auch delta -Natriumdisilikate Na₂Si₂O₅*yH₂O bevorzugt.

Einsetzbar sind auch amorphe Natriumsilikate mit einem Modul Na₂O: SiO₂ von 1: 2 bis 1: 3,3, vorzugsweise von 1:2 bis 1: 2,8 und insbesondere von 1:2 bis 1: 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharte Beugungsmaxima liefern. Dies ist so zu interpretieren, dass die Produkte mikrokristalline Bereiche der Größe 10 bis einige Hundert nm aufweisen, wobei Werte bis maximal 50 nm und insbesondere bis maximal 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Ein verwendbarer feinkristalliner synthetischer und gebundenes Wasser enthaltender Zeolith ist vorzugsweise Zeolith A und/oder P. Als Zeolith P wird Zeolith MAP TM (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma SASOL unter dem Markennamen VEGOBOND AX(R) vertrieben wird und durch die Formel

nNa2O*(1-n)K₂O*Al₂O₃.(2-2,5)*SiO₂.(3,5-5,5)*H₂O,

beschrieben werden kann, entspricht. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Messmethode: Coulter Counter) auf und enthalten vorzugsweise 18 bis 22 Gew.-%, insbesondere 20 bis 22 Gew.% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Als Builder sind organische Co-builder geeignet, insbesondere Polycarboxylate/Polycarbonsäuren, polymere Polycarboxylate, Asparaginsäure, Polyacetale, Dextrine, sowie Phosphonate.

Polymere Polycarboxylate sind beispielsweise die Alkalimetallsalze der Polyacrylsäure oder der Polymethacrylsäure, beispielsweise solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsaeure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsaeure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weissdextrine mit höheren Molmassen im Bereich von 2000 bis 30000 g/mol. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C6 des Saccharidrings oxidiertes Produkt kann besonders vorteilhaft sein.

Ein bevorzugtes Dextrin ist in der britischen Patentanmeldung GB 9,419,091 B1 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP 032202 A, EP 0427349 A, EP 0472042 A und EP 0542496 A sowie den internationalen Patentanmeldungen WO 1992/018542 A, WO 1993/008251 A, WO 1994/ 028030 A, WO 1995/007303 A, WO 1995/012619 A und WO 1995/020608 A bekannt. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4,524,009, US 4,639 325, in der europäischen Patentanmeldung EP 0150930 A und der japanischen Patentanmeldung JP 1993/339896 A beschrieben werden.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Co-builder werden beispielsweise in der internationalen Patentanmeldung WO 1995/020029 A beschrieben.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Wasch- und Reinigungsmittel auch Bleiche enthalten, bevorzugt sein Aminoalkan-phosphonate, insbesondere DTPMP, einzusetzen, oder Mischungen aus den genannten Phosphonaten zur Herstellung der Mittel zu verwenden. Darüber hinaus können alle Verbindungen, die in der Lage sind, Komplexe mit Erdalkaliionen auszubilden, als Co-builder eingesetzt werden.

### Bleichmittel und Bleichkatalysatoren

Als Bleichmittel, die verkapselt werden können sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure zu nennen. Um beim Waschen bei Temperaturen von 60 °C und darunter eine verbesserte Bleichwirkung zu erreichen, können Bleichaktivatoren in die Wasch- und Reinigungsmittel eingearbeitet werden. Als Bleichaktivatoren können Verbindungen, die unter Perhydrolysebedingungen aliphatische Peroxocarbonsäuren mit vorzugsweise 1bis 10 C-Atomen, insbesondere 2 bis 4 C-Atomen, und/oder gegebenenfalls substituierte Perbenzoesäure ergeben, eingesetzt werden. Geeignet sind Substanzen, die O-und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin(DADHT), acylierte Glykolurile, insbesondere Tetraacetylglykoluril (TAGU), N-Acylimide,insbesondere N-Nonanoylsuccinimid (NOSI), acylierte Phenolsulfonate, insbesonderen-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran. Zusätzlich zu den konventionellen Bleichaktivatoren oder an deren Stelle können auch sogenannte Bleichkatalysatoren in die Textilbehandlungsmittel eingearbeitet werden. Bei diesen Stoffen handelt es sich um bleichverstärkende Übergangsmetallsalze bzw. Übergangsmetallkomplexe wie beispielsweise Mn-,Fe-, Co-, Ru- oder Mo-Salenkomplexe oder -carbonylkomplexe. Auch Mn-, Fe-, Co-,Ru-, Mo-, Ti-, V- und Cu-Komplexe mit stickstoffhaltigen Tripod-Liganden sowie Co-, Fe-, Cu- und Ru-Aminkomplexe sind als Bleichkatalysatoren verwendbar.

### Verdickungsmittel

Ein flüssiges Wasch- und Reinigungsmittel kann ein Verdickungsmittel enthalten. Das Verdickungsmittel kann ebenfalls erfindungsgemäß verkapselt werden und umfasst beispielsweise Polyacrylat-Verdicker, Xanthan Gum, Gellan Gum, Guarkernmehl, Alginat, Carrageenan, Carboxymethylcellulose, Bentonite, Wellan Gum, Johannisbrotkernmehl, Agar-Agar, Tragant, Gummi arabicum, Pektin, Polyose, Stärke, Dextrin, Gelatine und Casein. Aber auch abgewandelte Naturstoffe wie modifizierten Stärken und Cellulosen, beispielhaft seien hier Carboxymethylcellulose und andere Celluloseether, Hydroxyethyl- und -propylcellulose sowie Kernmehlether genannt, können als Verdickungsmittel eingesetzt werden.

Zu den Polyacryl- und Polymethacryl-Verdickern zählen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients "der "The Cosmetic, Toiletry and Fragrance Association (CTFA)": Carbomer),die auch als Carboxyvinylpolymere bezeichnet werden. Solche Polyacrylsäuren sind u.a. von der Fa. 3V Sigma unter dem Handelsnamen Polygel^{®},z.B. Polygel DA, und von der Fa. B.F. Goodrich unter dem Handelsnamen Carbopol^{®} erhältlich, z.B. Carbopol 940 (Molekulargewicht ca. 4.000.000 g/mol), Carbopol 941 (Molekulargewicht ca. 1.250.000 g/mol) oder Carbopol 934 (Molekulargewicht ca. 3.000.000 g/mol).Weiterhin fallen darunter folgende Acrylsäure-Copolymere: (i) Copolymere von zwei oder mehr Monomeren aus der Gruppe der Acrylsäure, Methacrylsäure und ihrer einfachen, vorzugsweise mit C1-4-Alkanolen gebildeten, Ester (INCI AcrylatesCopolymer), zu denen etwa die Copolymere von Methacrylsäure, Butylacrylat und Methylmethacrylat (CAS-Bezeichnung gemäß Chemical Abstracts Service: 25035-69-2)oder von Butylacrylat und Methylmethacrylat (CAS 25852-37-3) gehören und die beispielsweise von der Fa. Rohm and Haas unter den Handelsnamen Aculyn^{®} und Acusol^{®} sowie von der Firma Degussa (Goldschmidt) unter dem Handelsnamen Tego^{®} Polymer erhältlich sind, z.B. die anionischen nicht assoziativen Polymere Aculyn 22, Aculyn 28, Aculyn 33 (vernetzt), Acusol 810,Acusol 820, Acusol 823 und Acusol 830 (CAS 25852-37-3); (ii) vernetzte hochmolekulare Acrylsäurecopolymere, zu denen etwa die mit einem Allylether der Saccharose oder des Pentaerythrits vernetzten Copolymere von C10-30-Alkylacrylaten mit einem oder mehreren Monomeren aus der Gruppe der Acrylsäure, Methacrylsäure und ihrer einfachen, vorzugsweise mit C1-4-Alkanolen gebildeten, Ester (INCI Acrylates/C10-30Alkyl Acrylate Crosspolymer) gehören und die beispielsweise von der Fa. B.F. Goodrich unter dem Handelsnamen Carbopol^{®} erhältlich sind, z.B. das hydrophobierte Carbopol ETD 2623 und Carbopol 1382 (INCI Acrylates/C10-30Alkyl Acrylate Crosspolymer) sowie Carbopol Aqua 30 (früher Carbopol EX 473).

Ein weiteres bevorzugt einzusetzendes polymeres Verdickungsmittel ist Xanthan Gum, ein mikrobielles anionisches Heteropolysaccharid, das von Xanthomonascampestris und einigen anderen Species unter aeroben Bedingungen produziert wird und eine Molmasse von 2 bis 15 Millionen g/mol aufweist. Xanthan wird aus einer Kette mit beta-1,4-gebundener Glucose (Cellulose) mit Seitenketten gebildet. Die Struktur der Untergruppen besteht aus Glucose, Mannose, Glucuronsaeure,Acetat und Pyruvat, wobei die Anzahl der Pyruvat-Einheiten die Viskosität des Xanthan Gums bestimmt. Als Verdickungsmittel kommt insbesondere auch ein Fettalkohol in Frage. Fettalkohole können verzweigt oder nichtverzweigt sowie nativen Ursprungs oder petrochemischen Ursprungs sein. Bevorzugte Fettalkohole haben eine C-Kettenlänge von 10 bis 20 C-Atomen, bevorzugt 12 bis 18. Bevorzugt werden Mischungen unterschiedlicher C-Kettenlängen, wie Talgfettalkohol oder Kokosfettalkohol, eingesetzt. Beispiele sind Lorol^{®} Spezial (C12-14-ROH) oder Lorol^{®} Technisch(C12-18-ROH) (beide ex Cognis). Bevorzugte flüssige Wasch- und Reinigungsmittel enthalten bezogen auf das gesamte Mittel 0.01 bis 3 Gew.% und vorzugsweise 0.1 bis1 Gew.% Verdickungsmittel. Die Menge an eingesetztem Verdickungsmittel ist dabei abhängig von der Art des Verdickungsmittels und dem gewünschten Grad der Verdickung.

### Enzyme

Die Wasch- und Reinigungsmitteln können Enzyme in verkapselter Form und/oder direkt in den Wasch- und Reinigungsmittel enthalten. Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, beta-Glucanasen, Oxidasen, Peroxidasen, Perhydrolasen und/oder Laccasen und Gemische der genannten Enzyme in Frage. Alle diese Hydrolasen tragen in der Wäsche zur Entfernung von Verfleckungen wie Protein-, fett- oder stärkehaltigen Verfleckungen und Vergrauungen bei. Cellulasen und andere Glykosylhydrolasenkoennen darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicolainsolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cellobiohydrolasen, Endoglucanasen und p-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme, der Enzymflüssigformulierung(en) oder der Enzymgranulate direkt in Wasch- und Reinigungsmittel kann beispielsweise etwa 0,01 bis 5 Gew.%, vorzugsweise 0.12 bis etwa 2.5 Gew.% betragen.

Es kann, beispielsweise bei speziellen Wasch- und Reinigungsmitteln für Konsumenten mit Allergien, aber auch bevorzugt sein, dass das Wasch- und Reinigungsmittel keine Enzyme enthält.

### Elektrolyte

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Wasch- und Reinigungsmitteln bevorzugt. Der Anteil an Elektrolyten in den Wasch- und Reinigungsmittel beträgt üblicherweise 0,1 bis 5 Gew.-%.

### Optische Aufheller

Optische Aufheller (sog. "Weisstöner") können den Wasch- und Reinigungsmitteln zugesetzt werden, um Vergrauungen und Vergilbungen der behandelten textilen Flächengebilde zu beseitigen. Diese Stoffe ziehen auf die Faser auf und bewirken eine Aufhellung und vorgetäuschte Bleichwirkung, indem sie unsichtbare Ultraviolettstrahlung in sichtbares längenwelliges Lichtumwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiss ergibt. Geeignete Verbindungen, die erfindungsgemäß verkapselt werden können, stammen beispielsweiseaus den Substanzklassen der 4,4'-Diamino-2,2'-stilbendisulfonsaeuren (Flavonsäuren), 4,4'-Distyryl-biphenylen, Methylumbelliferone, Cumarine, Dihydrochinolinone, 1,3-Diarylpyrazoline,Naphthalsaeureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0 % und 0.3 Gew.%, bezogen auf das fertige Wasch- und Reinigungsmittel, eingesetzt.

### Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise Leim, Gelatine, Salze von Ethersulfonsäuren der Stärke oder der Cellulose oder Salze von sauren Schwefelsäureestern der Cellulose oder der Stärke.

### Knitterschutzmittel

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- und Reinigungsmittel synthetische Knitterschutzmittel, die erfindungsgemäß verkapselt sind, enthalten. Hierzu zählen beispielsweise synthetische Produkte auf der Basis von Fettsäuren, Fettsäureestern, Fettsäureamiden, -alkylolestern,-alkylolamiden oder Fettalkoholen, die meist mit Ethylenoxid umgesetzt sind, oder Produkte auf der Basis von Lecithin oder modifizierter Phosphorsäureester.

### Antimikrobielle Wirkstoffe

Zur Bekämpfung von Mikroorganismen können die Wasch- und Reinigungsmitteln verkapselte antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppensind beispielsweise Benzalkoniumchloride, Alkylarylsulfonate, Halogenphenole und Phenolmercuriacetat.

### Antioxidantien

Um unerwünschte, durch Sauerstoffeinwirkung und andere oxidative Prozesse verursachte Veränderungen an den Wasch- und Reinigungsmitteln und/oder den behandelten textilen Flächengebilden zu verhindern, können die Wasch- und Reinigungsmittel Antioxidantien, die erfindungsgemäß verkapselt werden, enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

### Schauminhibitoren

Zur Verbesserung der Wiederbenetzbarkeit der behandelten textilen Flächengebilde und zur Erleichterung des Bügelns der behandelten textilen Flächengebilde können in den Textilbehandlungsmitteln beispielsweise Silikonderivate eingesetzt werden. Diese verbessern zusätzlich das Ausspülverhalten der Wasch- und Reinigungsmittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H-und/oder Si-Cl-Bindungen aufweisen. Die Viskositäten der bevorzugten Silikone liegen bei 25 °C im Bereich zwischen 100 und 100.000 mPas, wobei die Silikone in Mengen zwischen 0.2 und 5 Gew.%, bezogen auf das gesamte Wasch- und Reinigungsmittel eingesetzt werden können.

### UV-Absorber

Schließlich können die Wasch- und Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, können erfindungsgemäß verkapselt werden und sind beispielsweise die durch strahlungslose Deaktivierung wirksamen Verbindungen und Derivate des Benzophenons mit Substituenten in 2-und/oder4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

### Schwermetallkomplexbildner

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner, die erfindungsgemäß verkapselt werden, sind beispielsweise die Alkalisalze der Ethylendiamintetraessigsäure (EDTA) oder der Nitrilotriessigsäure (NTA) sowie Alkalimetallsalze von anionischen Polyelektrolyten wie Polymaleaten und Polysulfonaten. Eine bevorzugte Klasse von Komplexbildnern sind die Phosphonate, die in bevorzugten Textilbehandlungsmitteln in Mengen von 0.01 bis 2.5 Gew.-%, vorzugsweise 0.02 bis 2 Gew.% und insbesondere von 0.03 bis 1.5 Gew.% enthalten sind. Zu diesen bevorzugten Verbindungen zählen insbesondere Organophosphonate wie beispielsweise1-Hydroxyethan-1,1-diphosphonsäure (HEDP), Aminotri(methylenphosphonsäure) (ATMP), Diethylentriaminpenta(methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

### KOSMETISCHE ZUBEREITUNGEN

Die erfindungsgemäßen Kapseln sind insbesondere geeignet, um Wirkstoffe und Substanzen in kosmetischen Produkten (speziell Körperpflegemittel) und/oder pharmazeutischen Mitteln einzubringen. Bevorzugt sind hierbei die Parfümöle, Aromen, Aromastoffe, Duftstoffe.

Vorzugsweise können therapeutische Wirkstoffe in den erfindungsgemäßen Kapseln eingekapselt werden. Vorzugsweise umfassen kosmetische Produkte und pharmazeutische Mittel eine Reihe von Hilfs- und Zusatzstoffen. Diese Hilfs- und Zusatzstoffe können, je nach Notwendigkeit, auch in die erfindungsgemäßen Kapseln eingekapselt werden. Die typischen Hilfs- und Zusatzstoffe, die in kosmetischen Produkte und/oder pharmazeutischen Mittel enthalten sein können und, die auch in den erfindungsgemäßen Kapseln verkapselt werden können, sind beispielsweise milde Tenside, Ölkörper, Emulgatoren, Perlglanzwachse, Kühlstoffe, Konsistenzgeber, Verdickungsmittel, Überfettungsmittel, Stabilisatoren, Polymere, Silikonverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen.

Insbesondere Wirkstoffe, wie Kühlwirkstoffe sind geeignet, um in die erfindungsgemäßen Kapseln eingekapselt zu werden. Vorteilhaft ist bei einer solchen Einkapselung, dass die Kühlung, z.B. bei Cremes, Pasten, Sprays etc. erst bei der Anwendung, also beim Verreiben auf der Haut, einsetzt. Bei einem solchen Einsatz, z.B. als After-Sun Creme oder After-Sun Sprays sind die erfindungsgemäßen Kapseln besonders geeignet.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, vezweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettketone, Fettaldehyde, Fettether und Fettcarbonate, die in Summe mindestens 24 Kohlenstoffatome aufweisen, speziell Lauron und Distearylether.

### Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

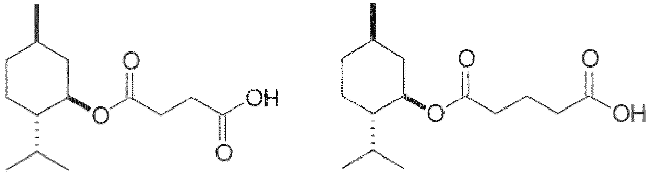

Beispiele für Anwendungen dieser Stoffe finden sich beispielsweise in den Druckschriften WO 2003 043431 (Unilever) oder EP 1332772 A1 (IFF).

Die nächste wichtige Gruppe, von im Sinne der Erfindung bevorzugten Mentholverbindungen, umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro-oder Nanopigmente eingesetzt. Vorzugsweise wird mikronisiertes Zinkoxid wie z.B. Z-COTE^{®} oder Z-COTE HP1 ^{®} verwendet.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- Konsistenzgeber.

Daneben können in Antitranspirantien übliche öllösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### Insektenrepellentien

Als Insekten-Repellentien kommen N,N-Diethyl-m-toluamid, 1,2-Pentandiol oder Ethyl Butylacetylaminopropionate in Frage.

### Inhaltsstoffe für Mund- und Zahnpflegemittel

Unter Mund- und Zahnpflegemittel sind Produkte zu verstehen, die der Mund- und Zahnreinigung und-pflege dienen. Beispiele hierfür sind Zahnpasten, Zahngele, und dergleichen.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 g/mol;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle, Aromen, Aromastoffe, Duftstoffe

Bevorzugt eingesetzte unverkapselte Duftstoffe bzw. Parfümöle, sind keinerlei Beschränkungen unterworfen. So können als Duftstoffe einzelne Riechstoffverbindungen, sowohl synthetische oder natürliche Verbindungen vom Typ der Ester, Ether, Aldehyde, Ketone, Alkohole, Kohlenwasserstoffe, Säuren, Kohlensäureester, aromatische Kohlenwasserstoffe, aliphatische Kohlenwasserstoffe, gesättigte und/oder ungesättigte Kohlenwasserstoffe und Mischungen daraus verwendet werden. Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschtem Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso jasmon, Methyl beta naphthyl keton, Moschus indanon, Tonalid/Moschus plus, Alpha-Damascon, Beta-Damascon, Delta-Damascon,lso-Damascon, Damascenon, Damarose, Methyl-dihydrojasmonat, Menthon, Carvon, Campher, Fenchon, Alphalonen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenylketon oder Methyl-cedrylon,Acetophenon, Methyl-acetophenon, Para-Methoxyacetophenon, Methyl-beta-naphtyl-keton,Benzyl-aceton, Benzophenon, Para-Hydroxyphenyl-butanon, Celery Keton oder Livescon,6-Isopropyldecahydro-2-naphton, Dimethyloctenon, Freskomenth, 4-(I-Ethoxyvinyl)-3,3,5,5,-tetramethyl-cyclohexanon,Methyl-heptenon, 2-(2-(4-Methyl-3-cyclohexen-1-yl)propyl)-cyclopentanon, 1-(p-Menthen-6(2)-yl)-1-propanon,4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon,4-Damascol, Dulcinyl or Cassion, Gelson, Hexa-Ion, Isocyclemon E, Methyl-cyclocitron, Methyl-Lavendel-keton, Orivon, Para-tert-butylcyclohexanon, Verdon, Delphon, Muscon, Neobutenon, Plicaton, Velouton, 2,4,4,7-Tetramethyl-oct-6-en-3-on, Tetrameran, Hedionund Gemischen davon. Bevorzugt können die Ketone ausgewählt sein aus AlphaDamascon, Delta Damascon, Iso Damascon, Carvon, Gamma-Methyl-ionon, Iso-E-Super,2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal,Ethylvanillin, Florhydral, Floralozon, Helional, Heliotropin, Hydroxycitronellal, Koavon, Laurinaldehyd, Canthoxal, Lyral, Lilial, Adoxal, Anisaldehyd, Cumal Methyl-nonyl-acetaldehyd, Citronellal, Citronellyloxyacetaldehyd, Cyclamenaldehyd, Bourgeonal, p,t-Bucinal, Phenylacetaldehyd, Undecylenaldehyd, Vanillin; 2,6,10-Trimethyl-9-undecenal, 3-Dodecen-I-al,alpha-n-Amylzimtaldehyd, 4-Methoxybenzaldehyd, Benzaldehyd, 3-(4-tert-Butylphenyl)-propanal,2-Methyl-3-(para-methoxyphenylpropanal), 2-Methyl-4-(2,6,6-trimethyl-2(1)-cyclohexen-1-yl)butanal,3-Phenyl-2-propenal, cis-/trans-3,7-Dimethyl-2,6-octadien-I-al, 3,7-Dimethyl-6-octen-I-al,[(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd,2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2. 1.0 (2,6)]-decylidene-8)-butanal;Octahydro-4,7-methano-IH-indenecarboxaldehyd; 3-Ethoxy-4-hydroxybenzaldehyd, para-Ethyl-alpha,alpha-dimethylhydrozimtaldehyd, alpha-Methyl-3,4-(methylenedioxy)-hydrozimtaldehyd,3,4-Methylenedioxybenzaldehyd, alpha-n-Hexylzimtaldehyd, m-Cymene-7-carboxaldehyd,alpha-Methylphenylacetaldehyd, 7-Hydroxy-3,7-dimethyl octanal, Undecenal, 2,4,6-Trimethyl-3-cyclohexene-I-carboxaldehyd,4-(3)(4-Methyl-3-pentenyl)-3-cyclohexen-carboxaldehyd, 1-Dodecanal, 2,4-Dimethylcyclohexene-3-carboxaldehyd,4-(4-Hydroxy-4-methyl pentyl)-3-cylohexene-I-carboxaldehyd, 7-Methoxy-3,7-dimethyloctan-1-al,2-Methyl undecanal, 2-Methyl decanal, 1-nonanal, 1-Octanal, 2,6,10-Trimethyl-5,9-undecadienal,2-Methyl-3-(4-tertbutyl)propanal, 3-(4-Ethylphenyl)-2,2-Dimethylpropanal, 3-(4-Methoxyphenyl)-2-methylpropanal,Methylnonylacetaldehyd, 2-Phenylpropan-1-al, 3-Phenylprop-2-en-1-al, 3-Phenyl-2-pentylprop-2-en-1-al,3-Phenyl-2-hexylprop-2-enal, 3-(4-Isopropylphenyl)-2-methylpropan-1-al, 3-(4-Ethylphenyl)-2,2-dimethylpropan-1-al,3-(4-tert-Butylphenyl)-2-methyl-propanal, 3-(3,4-Methylendioxyphenyl)-2-methylpropan-1-al,3-(4-Ethylphenyl)-2,2-dimethylpropanal, 3-(3-Isopropylphenyl)-butan-1-al, 2,6-Dimethylhept-5-en-1-al,Dihydrozimtaldehyd, 1-methyl-4-(4-methyl-3-pentenyl)-3-cyclohexene-1-carboxaldehyd,5 oder 6 Methoxyhexahydro-4,7-methanoindan-1 oder 2-carboxyaldehyd, 3,7-Dimethyloctan-1-al,1-Undecanal,10-Undecen-1-al, 4-Hydroxy-3-methoxybenzaldehyd, 1-Methyl-3-(4-methylpentyl)-3-cyclohexene-carboxyaldehyd,7-Hydroxy-3,7-dimethyl-octanal; trans-4-decenal, 2,6-Nonadienal, para-Tolylacetaldehyd;4-Methylphenylacetaldehyd, 2-Methyl-4-(2,6,6-trimethyl-1-cyclohexen-1-yl)-2-butenal,ortho-Methoxyzimtaldehyd, 3,5,6-Trimethyl-3-cyclohexenecarboxaldehyd, 3,7-Dimethyl-2-methylene-6-octenal,Phenoxyacetaldehyd; 5,9-Dimethyl-4,8-decadienal, Peony aldehyd (6,10-dimethyl-3-oxa-5,9-undecadien-1-al),Hexahydro-4,7-methanoindan-1-carboxaldehyd, Octanal, 2-Methyl octanal, alpha-Methyl-4-(I-methylethyl)benzeneacetaldehyd,6,6-Dimethyl-2-norpinene-2-propionaldehyd, para Methyl phenoxy acetaldehyd, 2-methyl-3-phenyl-2-propen-1-al,3,5,5-Trimethylhexanal, Hexahydro-8,8-dimethyl-2-naphthaldehyd, 3-Propyl-bicyclo[2.2.1]-hept-5-ene-2-carbaldehyd,9-Decenal, 3-Methyl-5-phenyl-1-pentanal, Methyl-nonyl acetaldehyd, 1-p-Menthene-q-carboxaldehyd,Citral oder Gemische davon, Lilial citral, 1-Decanal, n-Undecanal, n-Dodecanal, Florhydral, 2,4-Dimethyl-3-cyclohexen-1-carboxaldehyd 4-Methoxybenzaldehyde, 3-Methoxy-4-hydroxybenzaldehyde, 3-Ethoxy-4-hydroxybenz-aldehyde, 3,4-Methylendioxybenzaldehyd und 3,4-Dimethoxybenzaldehydund Gemische davon. Wie vorstehend beispielhaft ausgeführt, können die Duftaldehyde und Duftketone eine aliphatische, cycloaliphatische, aromatische, ethylenisch ungesättigte Struktur oder eine Kombination dieser Strukturen aufweisen. Es können fernerweitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander "Published 1960 and 1969 respectively, Reprinted2000 ISBN: Aroma Chemicals Vol. 1: 0-931710-37-5, Aroma Chemicals Vol. 2: 0-931710-38-3", verwiesen.

Geeignete Riechstoffverbindungen vom Typ der Ester sind beispielsweise Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-1-ol, 2,6-Dimethylheptan-2-ol, 2-Methylbutanol, 2-Methylpentanol,2-Phenoxyethanol, 2-Phenylpropanol, 2-tert-Butycyclohexanol, 3,5,5-Trimethylcyclohexanol,3-Hexanol, 3-Methyl-5-phenylpentanol, 3-Octanol, 1-Octen-3-ol, 3-Phenylpropanol,4-Heptenol, 4-Isopropylcyclohexanol, 4-tert-Butycyclohexanol, 6,8-Dimethyl-2-nonanol,6-Nonen-1-ol, 9-Decen-1-ol, alpha-Methylbenzylalkohol, alpha-Terpineol, Amylsalicylat,Benzylalkohol, Benzylsalicylat, beta-Terpineol, Butylsalicylat, Citronellol, Cyclohexylsalicylat, Decanol, Dihydromyrcenol, Dimethylbenzylcarbinol,Dimethylheptanol, Dimethyloctanol, Ethylsalicylat, Ethylvanilin, Anethol, Eugenol,Geraniol, Heptanol, Hexylsalicylat, Isoborneol, Isoeugenol, Isopulegol, Linalool,Menthol, Myrtenol, n-Hexanol, Nerol, Nonanol, Octanol, pa-ra-Menthan-7-ol, Phenylethylalkohol, Phenol, Phenylsalicylat, Tetrahydrogeraniol, Tetrahydrolinalool, Thymol, trans-2-cis-6-Nonadienol,trans-2-Nonen-1-ol, trans-2-Octenol, Undecanol, Vanillin, Zimtalkohol, wobei, wenn mehrere Duftstoffalkohole vorhanden sind, diese unabhängig voneinander ausgewählt sein können.

Duftstoffe bzw. Parfümöle können auch natürliche Riechstoffgemische sein, wie sie aus pflanzlichen Quellen zugänglich sind, z.B. Pine-, Citrus-, Jasmin-, Patchouli-, Rosen- oder Ylang-Ylang-öl. Ebenfallsgeeignet sind Muskateller-Salbeiöl, Kamillenöl, Nelkenöl, Melissenöl,Minzöl, Zimtblaetteröl, Lindenbluetenöl, Wacholderbeeröl, Vetiveröl, Olibanumöl, Galbanumöl und Labdanumöl sowie Orangenblütenöl, Neroliöl, Orangenschalenöl und Sandelholzöl. Ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limettenöl, Mandarinenöl, Melissenöl, Moschuskerneröl, Myrrhenöl, Nelkenöl, Neroliöl, Niaouliöl, Olibanumöl, Origanumöl, Palmarosaöl, Patchuliöl,Perubalsamöl, Petitgrainöl, Pfefferöl, Pfefferminzöl, Pimentöl, Pine-öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-öl, Ysop-öl, Zimtöl, Zimtblätteröl, Zitronellöl, Zitronenöl sowie Zypressenöl.

Ebenfalls als Duftstoff geeignet sind sogenannte Duftstoffvorläufer (Pro-Drug). Bei dieser Klasse von Verbindungen handelt es sich um Verbindungen, welche durch das Aufbrechen einer chemischen Bindung, beispielsweise durch Hydrolyse, ein erwünschtes Geruchs-und/oder Duftstoffmolekül freisetzt. Typischerweise wird zur Bildung eines Duftstoffvorläufers ein gewünschtes Duftstoffrohmaterial chemisch mit einem Träger, vorzugsweise einem geringfügig flüchtigen oder mäßig flüchtigen Träger, verbunden. Die Kombination führt zu einem weniger flüchtigen und stärker hydrophoben Duftstoffvorläufer mit verbesserter Anlagerung auf Stoffen. Der Duftstoff wird danach durch Aufbrechen der Bindung zwischen dem Duftstoffrohmaterial und dem Träger freigesetzt, beispielsweise durch eine Veränderung des pH-Werts (z. B. durch Transpiration beim Tragen), Luftfeuchtigkeit, Wärme und/oder Sonnenlicht während der Lagerung oder des Trocknens auf der Wäscheleine.

Das Duftstoffrohmaterial für Verwendung in Duftstoffvorläufern sind typischerweise gesättigte oder ungesättigte, flüchtige Verbindungen, die eine Alkohol-, einen- Aldehyd-und/oder eine Ketogruppe enthalten. Zu den hierin nützlichen Duftstoffrohmaterialien gehören jegliche wohlriechenden Substanzen oder Mischungen von Substanzen, die bereits oben beschrieben wurden. Besondere vorteilhafte, einsetzbare Duftstoffvorläufer gehorchen der Formel (III)

R-C(OR¹)(OR²)-OR³ (III)

worin R Wasserstoff, lineares C₁-C₈-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, substituiertes oder unsubstituiertes C₆-C₂₀-Aryl und Mischungen hiervon bedeutet; R¹, R² und R³ unabhängig lineares, verzweigtes oder substituiertes C₁-C₂₀-Alkyl; lineares, verzweigtes oder substituiertes C₂-C₂₀-Alkenyl; substituiertes oder unsubstituiertes, cyclisches C₃-C₂₀-Alkyl; substituiertes oder substituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Aryloxy; substituiertes oder unsubstituiertes C₆-C₄₀-Alkylenoxyaryl; C₆-C₄₀-Oxyalkylenaryl und Mischungen hiervon bedeuten. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer, sind Acetale oder Ketale, vorzugsweise gehorchend der Formel (IV)

R-C(R¹)(OR³)-OR2 (IV)

worin R lineares C₁-C₂₀-Alkyl, verzweigtes C₃-C₂₀-Alcyl, cyclisches C₆-C₂₀-Alkyl, verzweigtes cyclisches C₆-C₂₀-Alkyl, lineares C₂-C₂₀-Alkenyl, verzweigtes C₃-C₂₀-Alkenyl, cyclisches C6-C20-Alkenyl, verzweigtes cyclisches C₆-C₂₀-Alkenyl, substituiertes oder unsubstituiertes C₆-C₂₀-Aryl und Mischungen hiervon ist; R¹ Wasserstoff oder R ist; R² und R³ jeweils unabhängig voneinander gewählt sind aus der Gruppe, bestehend aus lineares C₁-C₂₀-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, C₆-C₂₀-Aryl, substituiertes C₇-C₂₀-Aryl und Mischungen hiervon. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer gehorchen der Formel (V)

R⁴O-C(OR¹)(OR³)-OR² (V)

worin R¹, R², R³ und R⁴ unabhängig voneinander lineares, verzweigtes oder substituiertes C₁-C₂₀-Alkyl; lineares, verzweigtes oder subsituiertes C₂-C₂₀-Alkenyl; substituiertes oder unsubstituiertes, cyclisches C₅-C₂₀-Alkyl; substituiertes oder unsubstituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Aryloxy; substituiertes oder unsubstituiertes C₆-C₄₀-Alkylenoxyaryl; C₆-C₄₀-Oxyalkylenaryl; und Mischungen hiervon sind. Der Einsatz solcher Substanzen, insbesondere in (vorzugsweise wasserunlöslichen) Mikrokapseln, entspricht einer bevorzugten Ausführungsform der Erfindung.

Besonders bevorzugt ist es, wenn die eingesetzten Riechstoffe Kieselsäureester-Mischungen umfassen. Kieselsäureester werden beispielsweise durch die Formel (V)

R-(-O-Si (OR)₂-)ₙ-OR (V)

beschrieben, wobei R unabhängig voneinander ausgewählt ist aus der Gruppe, die H, die geradkettigen oder verzweigten, gesättigten oder ungesättigten, substituierten oder unsubstituierten C₁-C₆-Kohlenwasserstoffreste und die Duftstoffalkoholreste und/oder Biozidalkoholreste enthält, und m Werte aus dem Bereich 1 bis 20 und n Werte aus dem Bereich 2 bis 100 annimmt. Vorzugsweise enthalten die Kieselsäureester der Formeln zumindest einen Duftstoffalkoholrest und/oder Biozidalkoholrest.

Die Kieselsäureester-Mischungen können verkapselt, aber auch unverkapselt zum Einsatz kommen. Die Anwesenheit von Kieselsäureester-Mischungen führt oftmals dazu, dass der erzielbare Dufteindruck, sowohl was Gefallen als auch Intensität anbetrifft, noch weiter verbessert werden kann. Der Dufteindruck ist nicht nur qualitativ, d. h. das Gefallen anbetreffend, besser, sondern halt auch länger an.

Die Kieselsäureester-Mischungen können auch in den Mikrokapseln enthalten sein. Wenn die Kieselsäureester-Mischungen in den Mikrokapseln vorzugsweise mindestens 2 Gew.- % der gesamten verkapselten Riechstoffmenge ausmachen, Gew.- % bezogen auf die Menge der verkapselten Riechstoffe, so liegt eine bevorzugte Ausführungsform der Erfindung vor, welche eine weitere Verbesserung des angestrebten Wohlgeruchseffektes nach dem Trocknen bewirkt.

Besonders geeignete Duftstoffvorläufer sind Reaktionsprodukte von Verbindungen, die mindestens eine primäre und/oder sekundäre Amingruppe umfassen, beispielsweise einem aminofunktionellen Polymer, insbesondere einem aminofunktionellen Silikon, und einem Duftstoffbestandteil, der aus Keton, Aldehyd und Mischungen davon ausgewählt ist. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Aromastoffe umfassen beispielsweise: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Bevorzugt werden Mischungen verschiedener Duftstoffe (aus den verschiedenen oben genannten Duftstoffklassen) verwendet, die gemeinsam eine ansprechende Duftnote erzeugen. In diesem Fall ist die Gesamtmenge des mindestens einen Duftstoffs die Menge aller Duftstoffe in der Mischung zusammen bezogen auf die Gesamtmenge des Mittels.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.1.42051), Indigotin (C.1.73015), Chlorophyllin (C.1.75810), Chinolingelb (C.I.47005), Titandioxid (C.1.77891), Indanthrenblau RS (C.I. 69800) und Krapplack (C.l.58000). Als Lumineszenzfarbstoff kann auch Luminol enthalten sein. Diese Farbtoffe werden üblicherweise in Konzentrationen von 0,001 bis 0,1 Gew.-%, bezogen auf die gesamte Mischung, eingesetzt.

In bevorzugten Ausführungsformen sind in den erfindungsgemäßen Kapseln Inhaltsstoffe von Klebstoffen und Agrochemikalien verkapselt.

### BEISPIELE

Die vorliegende Erfindung wird unter Bezugnahme auf die nachstehenden Beispiele leichter zu verstehen sein. Jedoch dienen diese Beispiele lediglich zur Veranschaulichung der Erfindung und können nicht als einschränkend in Bezug auf den Schutzbereich der Erfindung ausgelegt werden.

### Beispiel 1

### Herstellung von Mikrokapseln

Es wurden Mikrokapseln hergestellt, wobei einmal Resorcinol (KI, nicht erfindungsgemäß), dann 4-Aminophenol (KII) und danach 3-Aminophenol (KIII) bei der Aushärtung eingesetzt wurden. Hierbei wird ein wässriges Gemisch aus 31,5g Lupasol PA 140 und 36,56g Lucaroll SD gerührt und auf 35°C erwärmt. Anschließend wurde 180g eines Duftöls dazugegeben, weitergerührt und für weitere 30 min. emulgiert und die Teilchengröße entsprechend eingestellt. Dann wird solange Ameisensäure zugesetzt, bis das Reaktionsgemisch einen pH-Wert von 3,0-3,7 aufweist. Danach wird das Gemisch langsam auf 60°C erwärmt (1000U/min) und eine Melamin-Dispersion zugegeben. Anschließend wird 1h weitergerührt und danach auf 80°C erwärmt. Zum Reaktionsgemisch wird eine wässrige Lösung eines Aminophenols zugegeben (8,4g 3-Aminophenol und 42g Wasser) und 1 h bei 80°C gerührt. Der pH-Wert wird anschließend kontrolliert und eine wässrige Harnstofflösung zugefügt und bei 800-900 U/Min, 1h bei 80°C gerührt. Anschließend wird abgekühlt, ggf. mit Natronlauge und einem Verdicker nachbehandelt.

### Beispiel 2

### Stabilität und Rückhaltevermögen der Kapseln

Die Stabilität der in Beispiel 1 hergestellten Kapseln wurde bestimmt indem die Kapseln in eine Weichspülerformulierung (ca. 15% Esterquat) in einer Konzentration von 1 % eingearbeitet wurden und diese Mischung dann bei 45°C gelagert wurde. Anschließend wurde mit Hilfe von GC-Headspace-Messungen die Konzentration der in die Weichspülerformulierung eindiffundierten Riechstoffe bestimmt werden. Mit Hilfe dieser Ergebnisse wurde dann der Restanteil des noch in der Kapsel vorhandenen Parfümöls berechnet. Die Ergebnisse sind in der **Tabelle 1** wiedergegeben.

**Tabelle 1**

| Ergebnisse Stabilität (%) und Rückhaltevermögen | | | | | | |
|---|---|---|---|---|---|---|
| **Beurteilung nach** | **frisch** | **1 w** | **4w** | **8 w** | **12 w** | **Formaldehydkonzentration [ppm]** |
| Standard, 100% WB | 100 | 94 | 71 | 32 | 0 | 460 |
| Standard ohne Harnstoff, 100% WB | 100 | 97 | 81 | 46 | 0 | 1890 |
| Resorcin, 9,0g nach Melamin | 99 | 97 | 88 | 69 | 54 | 72 |
| 3-Aminophenol, 9,0g nach Melamin | 100 | 98 | 93 | 89 | 85 | 50 |
| 4-Aminophenol, 9,0g nach Melamin | 100 | 83 | 38 | 0 | 0 | 232 |
| Gemisch aus 3-Aminophenol und 4-Aminophenol (10:1), 9,0g nach Melamin | 100 | 85 | 60 | 40 | 32 | 105 |

### Beispiel 3

### Rückhaltevermögen der Kapseln

Das Rückhaltevermögen der in Beispiel 1 hergestellten Kapseln für einzelne Duftstoffverbindungen wurde ebenfalls bestimmt. Die Ergebnisse sind in **Tabelle 2** zusammengefasst:

**Tabelle 2**

| Rückhaltevermögen der Kapseln (Angaben in %) | | | | | |
|---|---|---|---|---|---|
| **Beurteilung nach** | **frisch** | **1 w** | **4 w** | **8 w** | **12 w** |
| ***Getesteter Duftstoff*** | ***Ethylmethylbutyrat-2*** | | | | |
| 3-Aminophenol | 100 | 91 | 70 | 49 | 48 |
| 4-Aminophenol | 100 | 33 | < 1 | < 1 | - |
| Resorcin | 100 | 96 | 60,6 | - | - |
| Standard * | 100 | 89 | - | - | - |
| Standard ** | 100 | 96 | 23 | - | - |

| ***Getesteter Duftstoff*** | ***Phenylethylalkohol*** | | | | |
|---|---|---|---|---|---|
| 3-Aminophenol | 92 | 66 | 66 | 66 | 50 |
| 4-Aminophenol | 78 | < 1 | < 1 | - | - |
| Resorcin | 88 | 3 | - | - | - |
| Standard * | 87 | - | - | - | - |
| Standard ** | 92 | - | - | - | - |

| ***Getesteter Duftstoff*** | ***p-Kresolmethylether*** | | | | |
|---|---|---|---|---|---|
| 3-Aminophenol | 100 | 72 | 30 | 30 | 39 |
| 4-Aminophenol | 100 | < 1 | < 1 | - | - |
| Resorcin | 100 | 71 | - | - | - |
| Standard * | 100 | 20 | - | - | - |
| Standard ** | 100 | 60 | - | - | - |

| ***Getesteter Duftstoff*** | ***Anisaldehyd*** | | | | |
|---|---|---|---|---|---|
| 3-Aminophenol | 100 | 79 | 78 | 77 | 60 |
| 4-Aminophenol | 91 | < 1 | - | - | - |
| Resorcin | 98 | 36 | - | - | - |
| Standard * | 95 | - | - | - | - |
| Standard ** | 98 | 27 | - | - | - |

| ***Getesteter Duftstoff*** | ***Phenylacetat*** | | | | |
|---|---|---|---|---|---|
| 3-Aminophenol | 100 | 96 | 79 | 72 | 58 |
| 4-Aminophenol | 100 | 34 | < 1 | - | - |
| Resorcin | 100 | 96 | 50 | - | - |
| Standard * | 100 | 90 | - | - | - |
| Standard ** | 100 | 95 | - | - | - |

Die verwendete Duftstoffmischung, die hierbei verwendet wurde, war gemäß **Tabelle 3** zusammengesetzt:

**Tabelle 3**

| Duftstoffmischung (Angaben in Gew.-%) | |
|---|---|
| **SUBSTANZ** | **ANTEIL** |
| AGRUMEX HC | 6,15 |
| ALDEHYD C 6 | 0,15 |
| ALDEHYD C 8 | 0,08 |
| ALDEHYD C 9 | 1,23 |
| ALDEHYD C11 UNDECYLENIC | 3,08 |
| ALDEHYD C12 MNA | 2,92 |
| AMAROCIT^{®} | 1,54 |
| AMBROCENIDE^{®} T 40 | 0,08 |
| ANETHOL SUPRA 21,5 CELSIUS | 0,31 |
| ANISALDEHYD REIN | 0,62 |
| BENZALDEHYD DD | 0,15 |
| BENZYLACETAT | 0,62 |
| CALONE | 0,08 |
| CASSIX 150 | 0,15 |
| CITRONELLYLPROPIONAT | 0,15 |
| CUMARIN | 0,62 |
| CYCLABUTE | 1,54 |
| CYMOL PARA SUPRA | 1,08 |
| DAMASCONE DELTA | 0,77 |
| DECENAL TRANS-2 | 0,31 |
| DYNASCONE | 0,15 |
| ETHYLBUTYRAT | 0,15 |
| ETHYLHEPTYLAT | 3,08 |
| ETHYLMETHYLBUTYRAT-2 | 4,62 |
| EUCALYPTOL NAT. | 1,85 |
| GALBASCONE | 0,15 |
| HELIOTROPIN/PIPERONAL | 0,15 |
| HERBAFLORAT | 3,08 |
| HEXYLZIMTALDEHYD ALPHA | 0,77 |
| IONON BETA | 0,23 |
| ISO E SUPER | 0,77 |
| ISOBORNYLACETAT | 18,00 |
| KAMPFER DL | 2,31 |
| KOAVONE | 0,31 |
| KRESOLMETHYLETHER PARA | 3,08 |
| LIGUSTRAL | 1,54 |
| MANZANATE | 0,38 |
| NECTARYL | 4,62 |
| NEROLIN YARA YARA KRIST. | 1,85 |
| ORANGENOEL BRAS. | 3,08 |
| ORYCLON^{®} SPEZIAL | 13,85 |
| PEONILE | 0,92 |
| PHARAONE 10% DPG | 0,15 |
| PHENYLETHYLACETAT | 0,15 |
| PHENYLETHYLALKOHOL | 2,31 |
| ROSENOXID L | 0,08 |
| STYROLYLACETAT | 2,31 |
| TERPINEN GAMMA | 0,23 |
| TERPINEOL REIN | 0,31 |
| TETRAHYDROLINALOOL | 6,15 |
| UNDECENAL TRANS-2 | 0,23 |
| VERTOCITRAL | 1,54 |

## Patentansprüche

1. Mikrokapseln, umfassend oder bestehend aus
(a) einem Kern, enthaltend mindestens ein, zwei oder mehrere Wirkstoffe, und
(b) einer Hülle,
wobei das Wandmaterial der Hülle geformt wird aus einem oder mehreren Aminoplasten, die gebildet werden aus
(i) mindestens einem Harnstoffderivat oder Melaminderivat,
(ii) einer Carbonylverbindung, und
(iii) mindestens einer Aminophenolkomponente der Formel (la) und/oder (Ib), 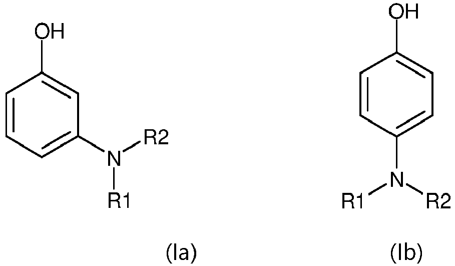
worin R1 und R2 unabhängig voneinander Wasserstoff, Methyl oder Ethyl darstellen
mit den Maßgaben, dass
- die Wirkstoffe Duftstoffe oder Parfümöle darstellen, von denen mindestens einer bei 25 °C flüssig sein muss, und
- die Harnstoffderivate oder Melaminderivate ausgewählt sind aus der Gruppe, die gebildet wird von 2,4,6-Triamino-1,3,5-triazin (Melamin), Tetrahyd-roimidazo[4,5-*d*]imidazole-2,5(1*H*,3*H*)-dion (Glycoluril), Benzoguanamin, Acetoguanamin, Adipo- und Glutaroguanamin oder einer Mischung daraus.

2. Mikrokapseln nach Anspruch 1, **dadurch gekennzeichnet, dass** die Aminophenolkomponente 3-Aminophenol ist.

3. Wasch- und Reinigungsmittel, kosmetische Zubereitungen oder Parfümzusammensetzungen, enthaltend Mikrokapseln nach Anspruch 1.

4. Agrochemikalien enthaltend Mikrokapseln nach Anspruch 1.

5. Klebstoffe enthaltend Mikrokapseln nach Anspruch 1.

## Claims

1. Microcapsules comprising or consisting of
(a) a core containing at least one, two or more active ingredients, and
(b) a shell,
wherein the wall material of the shell is formed from one or more aminoplasts formed from
(i) at least one urea derivative or melamine derivative,
(ii) a carbonyl compound, and
(iii) at least one aminophenol component represented by formula (Ia) and/or (Ib), 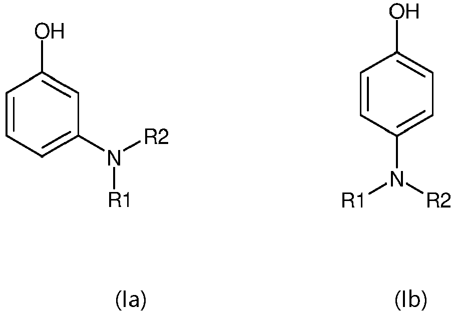
wherein R1 and R2 independently represent hydrogen, methyl or ethyl with the provisos that
- the active ingredients are perfumes or perfume oils, at least one of which must be liquid at 25°C, and
- the urea derivatives or melamine derivatives are selected from the group formed by 2,4,6-triamino-1,3,5-triazine (melamine), tetrahydroimidazo[4,5-d]imidazole-2,5(1H,3H)-dione (glycoluril), benzoguanamine, acetoguanamine, adipo- and glutaroguanamine or a mixture thereof.

2. Microcapsules according to claim 1, **characterized in that** the aminophenol component is 3-aminophenol.

3. Detergents, cosmetic preparations or perfume compositions containing microcapsules according to claim 1.

4. Agrochemicals containing microcapsules according to claim 1.

5. Adhesives containing microcapsules according to claim 1

## Revendications

1. Microcapsules comprenant ou constituées de
(a) un noyau contenant au moins une, deux ou plusieurs substances actives, et
(b) une coquille,
dans lequel le matériau de paroi de la coquille est formé d'un ou plusieurs aminoplastes formés à partir de
(i) au moins un dérivé d'urée ou un dérivé de mélamine,
(ii) un composé carbonyle, et
(iii) au moins un composant aminophénol de formule (la) et/ou (Ib), 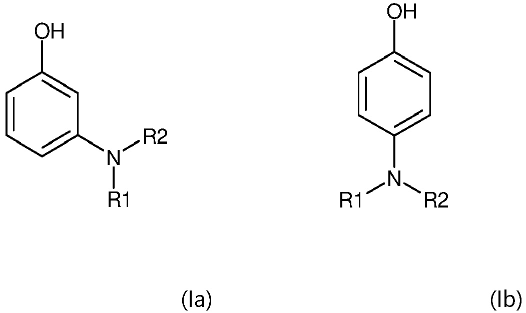
dans laquelle R1 et R2 sont indépendamment un hydrogène, un méthyle ou un éthyle
avec les conditions suivantes
- les principes actifs sont des parfums ou des huiles parfumées, dont au moins un doit être liquide à 25°C, et
- les dérivés d'urée ou de mélamine sont choisis dans le groupe formé par la 2,4,6-triamino-1,3,5-triazine (mélamine), la tétrahydroimidazo[4,5-d]imidazole-2,5(1*H*,3*H*)-dione (glycoluril), la benzoguanamine, l'acétoguanamine, l'adipo- et la glutaroguanamine ou un mélange de celles-ci.

2. Microcapsules selon la revendication 1, **caractérisées en ce que** le composant aminophénol est le 3-aminophénol.

3. Détergents, préparations cosmétiques ou compositions parfumées contenant des microcapsules selon la revendication 1.

4. Produits agrochimiques contenant des microcapsules selon la revendication 1.

5. Adhésifs contenant des microcapsules selon la revendication 1
